# EUROPEAN PATENT SPECIFICATION

(11) **EP 0 820 739 B1**
(45) Date of publication and mention of the grant of the patent: **23.06.2004**
(21) Application number: 97305475.2
(22) Date of filing: 22.07.1997
(51) Int. Cl.: A61F 2/38, A61F 2/00

(54) **Fastening system for a modular knee prosthesis**
Befestigungssystem für eine modulare Knieprothese
Système de fixation pour prothèse modulaire du genou

(30) Priority: 23.07.1996 US 685289; 14.08.1996 US 696495
(43) Date of publication of application: 28.01.1998
(73) Proprietor: DePuy Products, Inc., Warsaw, IN 46581 (US)
(72) Inventor: Coleran, Dennis P., Plainville, Massachusetts 02762 (US); Gabriel, Stefan M., Lakeville, Massachusetts 02347 (US)
(74) Representative: Fisher, Adrian John

(56) References cited:
- EP-A- 0 714 645
- EP-A- 0 781 535
- FR-A- 2 711 510
- US-A- 4 822 366
- US-A- 5 269 784

## Description

### Field of the Invention

This invention relates to joint prostheses, and more particularly to a medical fastening system for a modular knee joint prostheses employed during knee arthroplasty procedures.

### Background of the Invention

Knee arthroplasty is a well-known surgical procedure by which a diseased and/or damaged natural knee joint is replaced by a prosthetic knee joint. Typical knee prostheses include a tibial component, a femoral component, and a patellar component. The femoral component generally includes a pair of spaced apart condylar portions, the superior surfaces of which articulate with a portion of the tibial component. A femoral stem assembly, used to provide lateral stability to the replaced knee joint, seats within the medullary canal of a distal portion of a femur, and is typically coupled to the femoral component by specialized coupling devices, such as a collar and bolt. Some prosthetic knee joints include a structure known as a Morse taper post that extends from the inferior surface of the femoral component to mate with a femoral sleeve that is securable to the femoral stem assembly. See, for example, FR2711510.

The femoral sleeve, which helps to fill spaces at the opening of the medullary canal, can also provide for a modular assembly allowing a surgeon to select the most appropriate femoral stem from a selection of stems having different lengths and diameters for attachment to one of a selection of femoral components. This modular configuration significantly reduces the number of individual components that must be purchased, stocked, and used during a surgical procedure. Although the femoral stem, whatever its dimensions, is usually angled laterally with respect to the inferior surface of the femoral component and either off-set anteriorially/posterially or at a central location, it is sometimes desirable to orient the femoral stem perpendicularly with respect to the inferior surface. For example, depending on particular patient requirements, the femoral stem may need to be offset fore or aft with respect to the front of the femoral component. Similarly, the femoral stem may need to be angled varying degrees to the left or right with respect to the front of the femoral component. The Morse taper post, however, is integrally cast as a unitary and indivisible portion of the femoral component. Furthermore, there is a requirement for a range of sizes of the overall femoral component. Therefore, in order to accommodate all of the possible combinations of overall femoral component size, fore/neutral/aft positioning of the Morse taper post, and left/perpendicular/right angling of the Morse taper post, a doctor or hospital is required to maintain an undesirably substantial stock of knee prosthesis components. US-A-4822366 makes known a knee-prothesis having a modular femoral system. The femoral system has a femoral component with an inferior surface and a superior condylar-like surface, and a femoral stem structure anchored directly to the inferior surface. EP-A-0714645 also discloses a modular knee prothesis.

Despite the existence of knee joint prostheses having modular components, there remains a need for a modular knee joint prosthesis that has greater versatility to accommodate differing patient anatomy and joint conditions. It is thus an object of the invention to provide a modular knee prosthesis having greater versatility to accommodate different patient anatomy and joint conditions while maintaining a relatively low component count. It is another object of the invention to provide a modular knee prosthesis having components that are physiologically and geometrically compatible with different anatomical conditions. Still another object of the invention is to provide a modular knee prosthesis that is suitable for use in both right and left knee procedures. Other general and more specific objects of the invention will in part be apparent from the drawings and description that follow.

In EP-A-0781535 there is made known a medical fastening system having the features of the pre-characterizing part of claim 1, appended hereto.

### Summary of the Invention

The present invention relates to a modular knee joint prosthesis having improved versatility while reducing the overall component count. Components of the modular prosthesis of the invention are able to be used with both right and left side prostheses.

According to the present invention there is provided a medical fastening system for a modular knee prosthesis as set forth in claim 1 appended hereto.

In accordance with the present invention, a modular knee fastening system for a modular knee prosthesis includes a washer engagable with a bolt, and a femoral component so that a portion of the bolt shaft protrudes through an aperture in the washer and an aperture in the femoral component. The washer includes an aperture that is off-center or lobed to permit selective placement of the bolt with respect to the femoral component

The washer has an aperture alignable with at least a portion of the aperture in the femoral component. The washer engages the femoral component to inhibit movement of the washer through the aperture in the femoral component. A bolt engages the washer and an elongate shaft portion of the bolt protrudes from the femoral component through the aperture in the washer and the aperture in the femoral component to engage a Morse taper post or femoral stem. The configuration of the washer aperture, its location in the washer, and the orientation of the washer within the femoral component determine the fore and aft positioning of the Morse taper post or femoral stem. The Morse taper post or femoral stem can be provided with a canted base to angle the post or stem with respect to the femoral component.

### Brief Description of the Drawings

The foregoing and other objects, features and advantages of the invention will be apparent from the following description and the accompanying drawings, in which like reference characters refer to the same parts throughout the different views.
FIG. 1 is a cutaway exploded view of a modular knee prosthesis not according to the present invention that includes a right knee femoral component;
FIG. 2 is a perspective view of the femoral component of the modular knee prosthesis of FIG. 1;
FIG. 3 is a bottom perspective view of the femoral component of FIG. 2;
FIG. 4A is a side view of one embodiment of a securing bolt useful with the modular knee prosthesis of FIG. 1;
FIG. 4B is a top view of the securing bolt of FIG. 4A;
FIG. 5A is a side view of an altemate embodiment of a securing bolt useful with the modular knee prosthesis of FIG. 1;
FIG. 5B is a top view, from the shaft, of the securing bolt of FIG. 5A;
FIG. 6A is a side view of a collar useful with the modular knee prosthesis of FIG. 1;
FIG. 6B is a top view of the collar of FIG. 6A;
FIG. 6C is a cross-sectional view of the collar of FIG. 6A taken along line B-B of FIG.6B;
FIG. 7 is a cutaway exploded view of an a modular knee prosthesis not according to the present invention, wherein a femoral stem is directly mountable on a Morse taper post;
FIG. 8 is a cutaway view of an embodiment of the invention having a bolt and washer fastening system;
FIG. 9 is a side view of a washer in accordance with the invention;
FIG. 10 is a top view of a washer not in accordance with the invention;
FIG. 11 is a top view of an alternative embodiment of a washer in accordance with the invention;
FIG. 12 is a top view of an yet another embodiment of a washer in accordance with the invention;
FIG. 13 is an end view of the washer of FIG. 12 in association with a bolt in a first position;
FIG. 14 is a side view of the bolt and washer of FIG. 13;
FIG. 15 is an end view of the washer of FIG. 12 in association with a bolt in a second position; and
FIG. 16 is a side view of the bolt and washer of FIG. 15.

### Detailed Description of the Invention

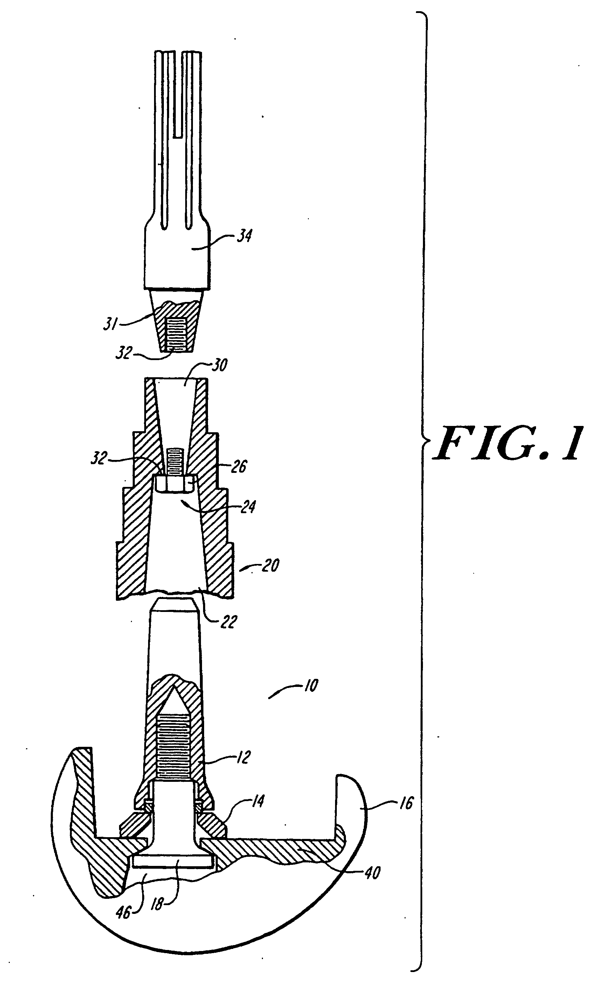

A modular knee prosthesis 10 of the invention may include a Morse taper post 12, a femoral component 16, and a securing bolt 18. FIG. 1 illustrates a modular knee prosthesis 10 not in accordance with the present invention. It includes a femoral component 16 adapted for a right knee. The Morse taper post 12, collar 14, and securing bolt 18 are suitable for use, without modification, in association with a femoral component adapted for a left knee.

A femoral sleeve 20, adapted for mating with the Morse taper post 12, includes a first end that defines a first cavity 22 for receiving the distal end portion of the Morse taper post. In the illustration, the first cavity 22 is tapered to provide a friction fit over the Morse taper post 12. A femoral sleeve stem bolt 24, having a head 26 and a shank 28 is positionable within the femoral sleeve 20. The shank 28 projects into a second cavity 30 defined in the second end of the femoral sleeve 20. In an exemplary embodiment, the femoral sleeve 20 includes a constriction or shoulder 32 that prevents the head 26 from entering into the second cavity 30 or otherwise anchors the femoral sleeve stem bolt 24 within the femoral sleeve 20. The femoral sleeve stem bolt 24 is adapted to engage a mating portion 32 of a femoral stem 34 selected from a group of femoral stems having different lengths and diameters. The illustrated femoral stem has a tapered end 31 that is receivable within the second cavity 30 of the femoral sleeve, which has a complimentary taper. In other embodiments of the invention, the Morse taper post is directly matable with a femoral stem or other component without a femoral sleeve.

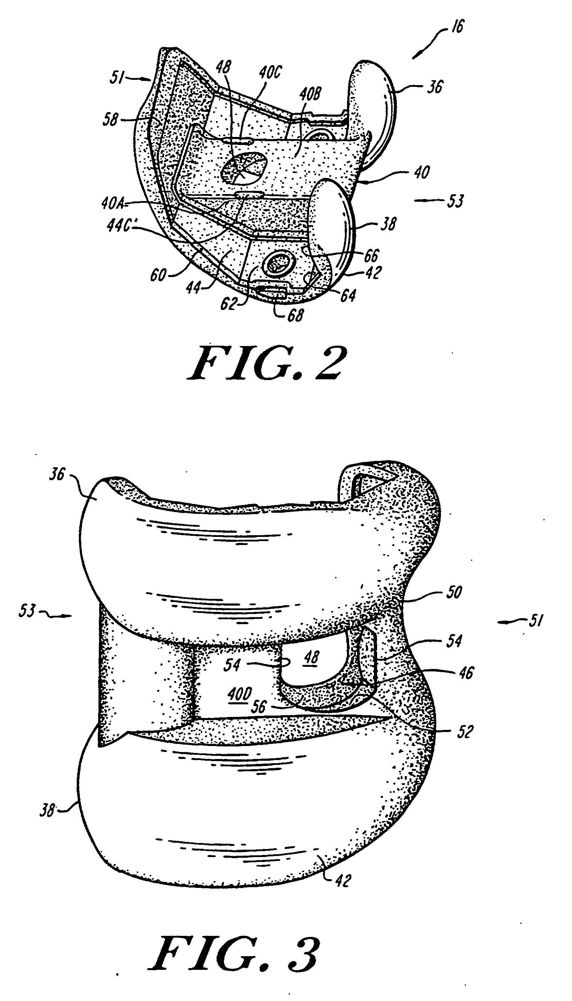

Referring to FIGS. 1 through 3, the femoral component 16 has a pair of condylar portions 36, 38 that are connected by an intercondylar region or boss 40. The femoral component 16 has a superior articulation surface 42 and an opposed inferior surface 44. Further, the femoral component 16 has a posterior side 53 and an anterior side 51. The anterior side 51 of the femoral component 16 includes a patellar groove 50, shown in FIG. 3, within which seats a patellar prosthetic component (not shown). The superior surfaces 42 of the curved condylar portions 36, 38 articulate with a prosthetic tibial component (not shown) mounted on the head of the tibia, in a manner well known to those of ordinary skill in the art.

The boss structure 40 has a pair of substantially vertical side walls 40A that are generally orthogonal to a top, inferior surface 40B. The top surface 40B preferably has formed thereon a pair of raised ridges 40C that constitute a collar anti-rotation element, as described in further detail below.

With reference to FIGS. 1 and 3, the boss 40 has a cavity 46 formed within a bottom superior surface 40D. An aperture 48 defined by the cavity 46 extends between the superior and inferior surfaces 42, 44, respectively, of the boss structure 40 and has a selected shape such that it can be elongated either in the anterior-posterior direction or the medial-lateral direction. The shape of the aperture can be elliptical, oval, spherical, or of any other suitable shape that allows a sufficient amount of translation of the securing bolt shaft when the bolt is mounted within the aperture.

In the illustrated embodiment, the cavity 46 has a pair of arcuate medial and lateral side walls 52, and a pair of substantially flat anterior and posterior side walls 54 that form a bolt anti-rotation mechanism, as described in further detail below. The cavity 46 further includes an end wall 56 that has a substantially spherical or rounded shape for seating a correspondingly shaped head of the securing bolt 18.

The inferior surface 44 of the condylar portions 36, 38 forms a series of integral surfaces that extend between the anterior and posterior sides of the femoral component. Referring to FIG. 2, the inferior surface of each condylar portion comprises a substantially vertical anterior surface 58, an anterior chamfer surface 60, a substantially horizontal surface 62, a posterior surface 64, and a substantially vertical posterior surface 66. The surface 62 of each condylar portion has an indentation 68 that extends partly into the inferior surface of each condylar portion. The indentation allows the surgeon to grasp and handle the femoral component via a suitable handling instrument. Those of ordinary skill in the art will recognize that the femoral component 16, boss 40, and condylar portions 36,38 can have a variety of shapes.

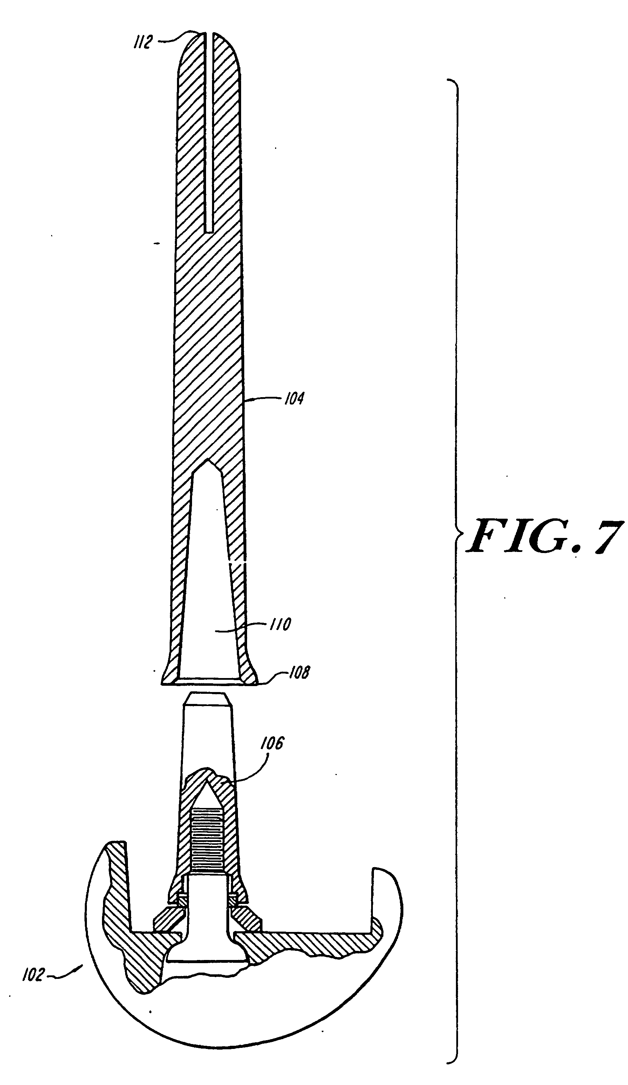

Referring now to FIG. 7, a modular knee prosthesis 102 is illustrated that does not include a femoral sleeve. In this embodiment, a femoral stem 104 is adapted for mating directly with a Morse taper post 106. More particularly, the femoral stem includes a first end 108 that defines a cavity 110 that is tapered to provide a friction fit over the Morse taper post 106. A second end 112 of the femoral stem is adapted for placement in a patient's medullary canal. In substantially all other respects, however, the remaining components of the modular knee prosthesis are identical to the components illustrated in Fig. 1.

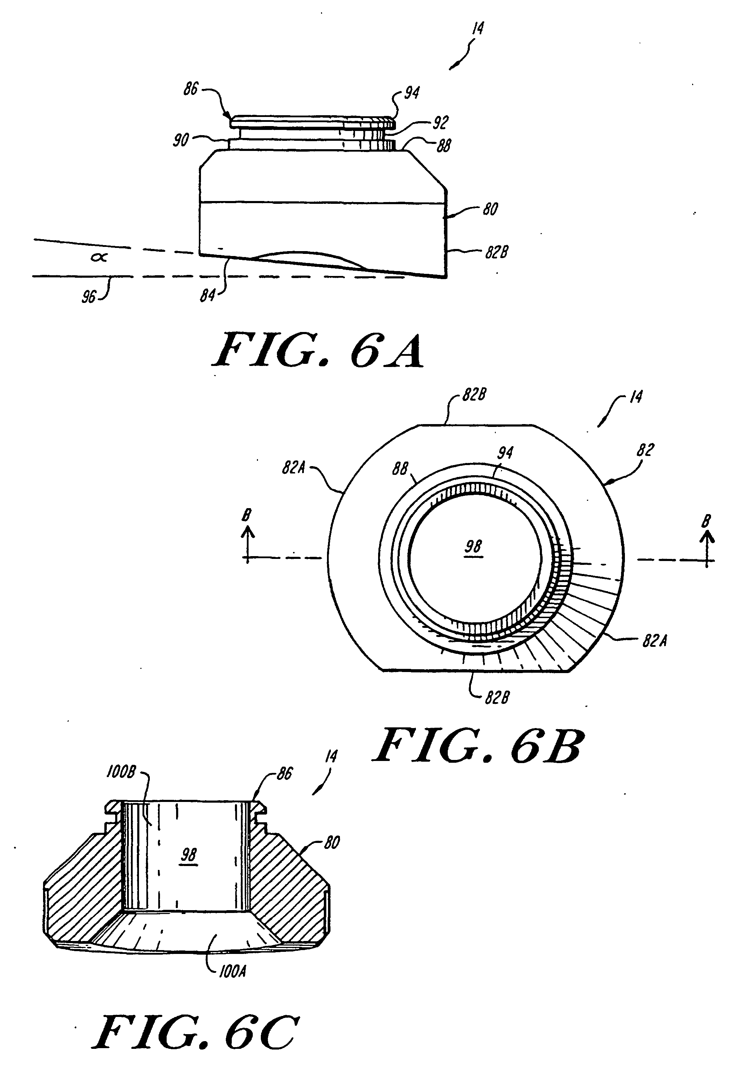

With respect to each of the embodiments shown in Figs 1 to 7, a modular collar 14 increases the adaptability of the modular knee prosthesis 10. However, the invention may include a Morse taper post that has features of the collar, such as a canted boss mounting surface, funnel-like portion, opposed arcuate sides, and opposed flat sides. As these configurations could preclude the Morse taper post from rotating during assembly, because its base is lodged between the raised ridges of the femoral component, a securing bolt can be provided that is rotatable with respect to the femoral component to urge the securing bolt and Morse taper post together.

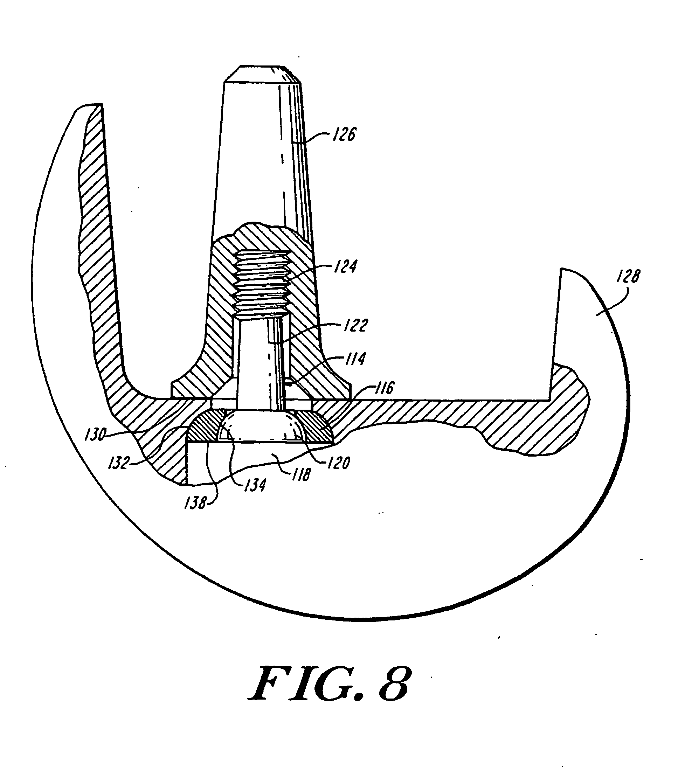

For example, FIG. 8 is a cutaway view of an embodiment, of the invention having a securing bolt and washer fastening system. It does not include a collar. In this embodiment, the securing bolt of FIGS. 1-6 is replaced by a bolt 114 and a washer 116, wherein the bolt and washer are rotatable with respect to each other and are collectively cooperative with the configuration of a boss cavity 118 to facilitate angulation and translation of the bolt as described above with respect to FIGS. 1-7. The bolt includes a head 120, a shank 122, and an engagement feature 124 such as threads. A supplemental component 126, such as a Morse taper post or femoral stem includes features, such as threads, that cooperate with the engagement feature 124 of the bolt 114 to allow the bolt to be firmly mated to the supplemental component and a femoral component 128. As a Morse taper post is illustrated in FIG. 8, the supplemental component 126 will be referred to as such during the descriptions that follow.

The lateral angulation of the Morse taper post 126 with respect to the femoral component 128 is determined by the cant of a boss mounting surface 130. In FIG. 8, the plane defined by the boss mounting surface 130 is substantially perpendicular to the longitudinal axis of the Morse taper post to provide a neutral or 0 degree orientation. In other embodiments, the boss mounting surface defines a plane that is not perpendicular to the longitudinal axis of the Morse taper post to provide a selected angulation to the right or left with respect to the front of the femoral component.

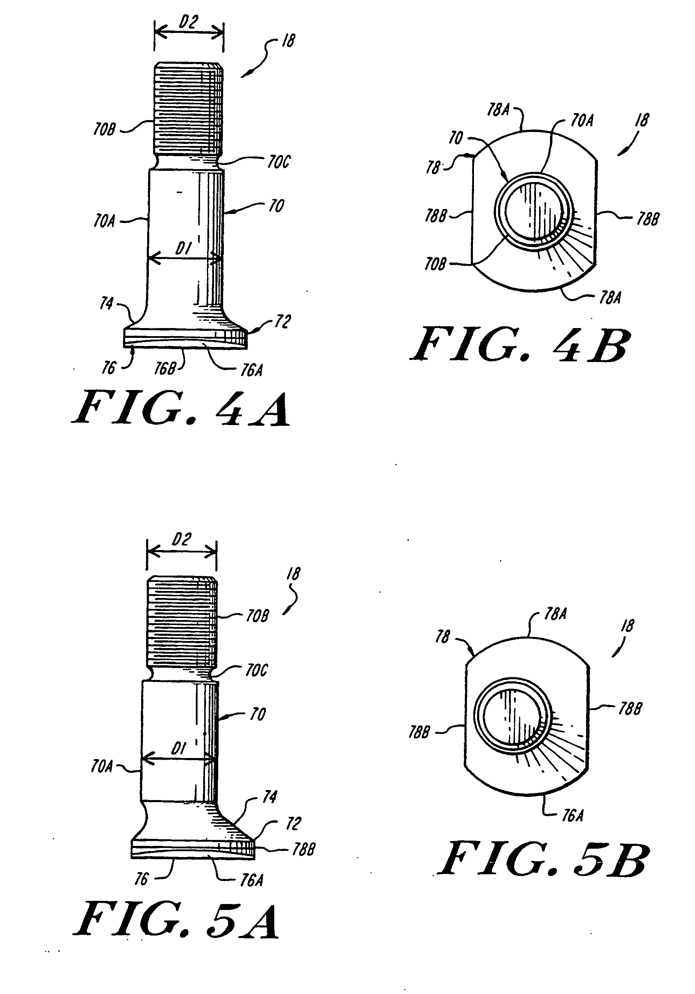

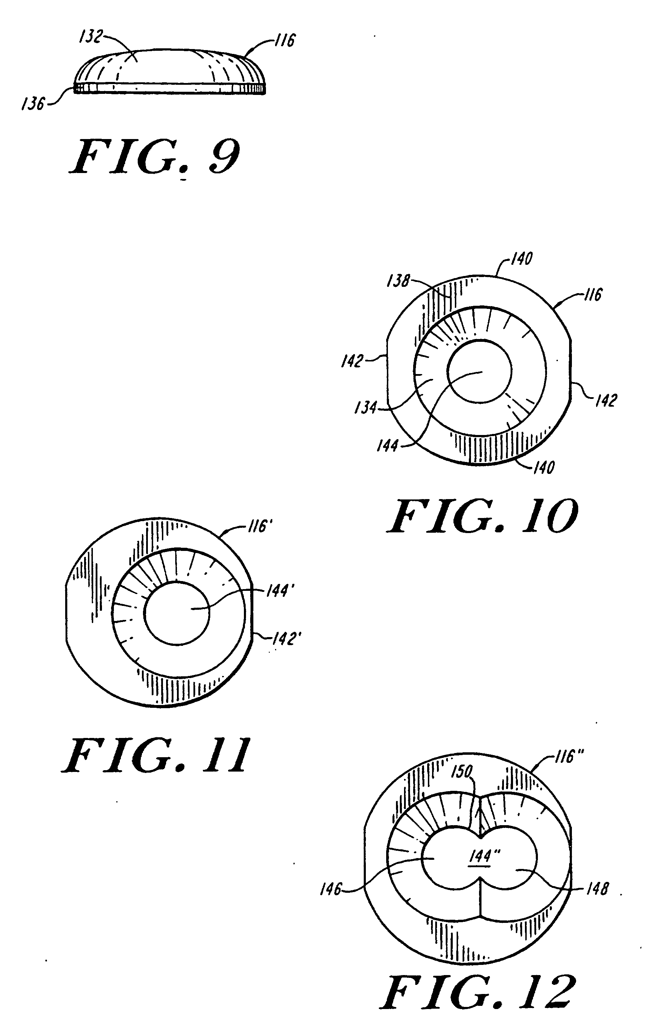

Positioning or translation of the bolt 114 fore and aft is accomplished by selection of an appropriate washer 116 as illustrated in FIGS. 9-16. Each of the illustrated washers 116 includes a spherical boss-engaging or inferior surface 132, a contoured bolt head-engaging or superior surface 134, a peripheral surface 136, a top surface 138, a pair of opposed, arcuate sides 140, and a pair of opposed substantially flat sides 142. The flat sides 142 matingly engage a flat side wall of the boss cavity 118 and cooperate therewith to secure the washer 116 within the cavity and prevent unwanted rotation of the washer in a manner similar to that described above with respect to FIG. 4B.

FIG. 11 illustrates a washer 116' in accordance with the invention wherein an aperture 144' is not at center of the washer, but is offset toward one of the substantially flat sides 142'. Thus, offset of the bolt 114 can be achieved with this washer by orienting the washer within the boss cavity 118 so that the aperture 144' is either closer to the front or the back of the femoral component 128.

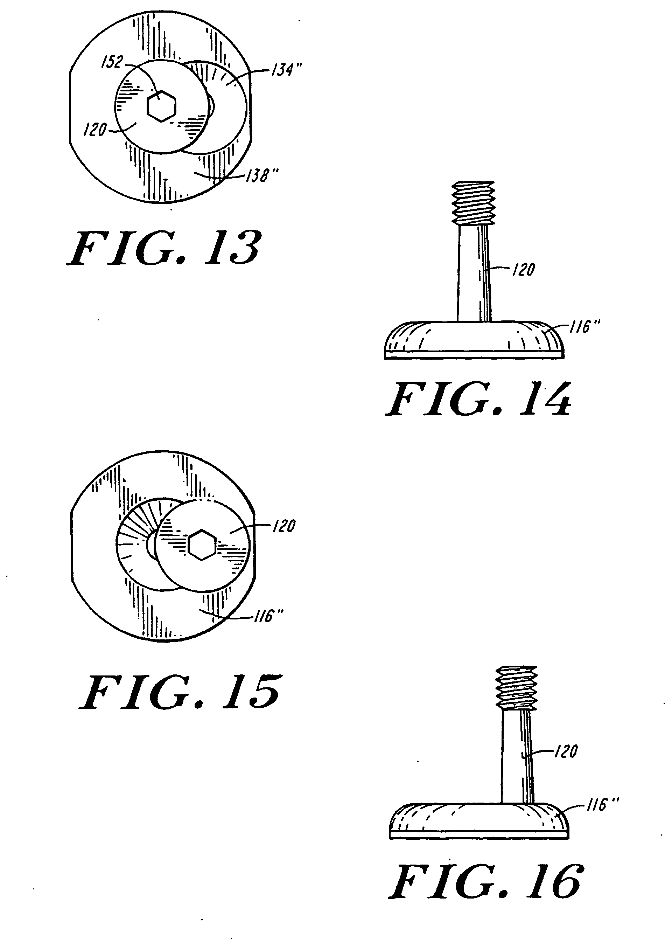

FIG. 12 illustrates an embodiment of the washer 116" having a double-lobed aperture 144", wherein each of the aperture lobes 146 and 148 is dimensioned to receive the bolt shank 122 therethrough. A neck portion 150 locally reduces the diameter of the aperture 144" and defines the first and second lobes 146, 148. At the neck portion 150, the aperture 144" has a smaller diameter than the bolt shank 122. However, the open configuration of the neck portion 150 allows a curved side portion of the bolt head or shank to extend into the principally unoccupied lobe as shown in FIGS. 13 and 15. This double-lobed configuration provides particular benefits in an application requiring a bolt to be positioned in either of a first or a second precisely defined location, but wherein the required bolt shank or head dimensions in association with the close proximity of the first location to the second location preclude the provision of two separate and distinct apertures. Additionally, a double-lobed configuration having a first lobe centrally located and an offset second lobe allows a single washer to be used in a kit to provide fore, neutral, and aft positioning of the bolt 120 by appropriate orientation of the washer within the femoral component and insertion of the bolt through one of the lobes.

FIG. 13 is an end view of the washer 116" of FIG. 12 in association with a bolt in a first, central position and FIG. 14 is a side view of the bolt and washer of FIG. 13. The bolt 120 is illustrated with a slot 152 having six flattened sides suitable for engaging a hex wrench; however, the bolt head can be provided with other configurations known to those skilled in the art to permit the bolt to be tightened with a tool or by hand. FIG. 15 is an end view of the washer of FIG. 12 in association with a bolt in a second, offset position and FIG. 16 is a side view of the bolt and washer of FIG. 14.

Thus, an exemplary kit may include a selection of washers, a single bolt, and a selection of Morse taper posts and/or femoral stems, and be assembled in the following manner. A Morse taper post having the desired angulation is selected and mounted on the top surface of the boss, and the flat sides of the Morse taper post are aligned between the raised ridges. A washer having the desired aperture location is selected and a bolt is inserted through the aperture. The washer is then inserted into the boss cavity from the underside of the boss and the bolt shank is passed through the boss aperture, such that the bolt shaft extends upwardly from the boss inferior surface. The spherical engaging surface of the washer mates with and engages the similarly configured end wall of the cavity and the sides of the washer engage the sides of the boss cavity to inhibit rotation of the washer. The selected shape of the washer and location of the aperture determines the offset of the bolt. The threads of the bolt engage the threads of the Morse taper post and the bolt is rotated to urge the bolt and Morse taper post together.

It will thus be seen that the invention efficiently attains the objects set forth above, among those made apparent from the preceding description. Since certain changes may be made in the above constructions without departing from the scope of the claims, it is intended that all matter contained in the above description or shown in the accompanying drawings be interpreted as illustrative and not in a limiting sense. It will also be understood that the inclusion in the claims of reference numerals from the Figures of drawings is for illustration and not meant to have a limiting effect or the scope of the claims.

## Claims

1. A medical fastening system for a modular knee prosthesis (102) comprising:
a femoral component (128) having a superior surface (42), an inferior surface (44), and an aperture (48) extending therebetween, the superior surface (42), in use, facing the tibia;
a washer (116) having a superior surface (134), an inferior surface (132), and an aperture (144) extending therebetween, the inferior surface (132) of the washer (116) being engagable with the superior surface (42) of the femoral component (128) to inhibit movement of the washer (116) through the aperture (48) in the femoral component (128); and
a bolt (114) including a head portion (120) engagable with the superior surface (134) of the washer (116) to inhibit movement of the bolt (114) through the aperture (144) in the washer (116), and an elongate shaft portion (122) extending from the head portion (120) of the bolt (114), the elongate shaft portion (122) having a length sufficient to protrude through the aperture (144) in the washer (116) and the aperture (48) in the femoral component (128) beyond the inferior surface (44) of the femoral component (128);
wherein:
the aperture (144) of the washer (116) is smaller than the aperture (48) in the femoral component (128);
the aperture (48) in the femoral component (128) is elongate and has an anterior portion and a posterior portion;
the washer (116) is matable with the femoral component (128) to position the aperture (144) of the washer (116) toward the anterior portion of the aperture (48) of the femoral component (128); and
the washer (116) is matable with the femoral component (128) to position the aperture (144) of the washer (116) toward the posterior portion of the aperture of the femoral component;
**characterised in that**:
the aperture (144) in the washer (116) is not in the center of the washer (116), the two aperture positions being achieved by changing the orientation of the washer (116).

2. The medical fastening system of claim 1, further comprising supplemental component (126) engagable with the elongate shaft portion (122) of the bolt (114).

3. The medical fastening system of claim 2, wherein the supplemental component 126) is a Morse taper post.

4. The medical fastening system of claim 3, wherein the Morse taper post (126) includes a longitudinal axis, a base defining an aperture therein for receiving the elongate shaft portion (122) of the bolt (114) therein and defining a plane that is perpendicular to the longitudinal axis, and a tapered distal end.

5. The medical fastening system of claim 3, wherein the Morse taper post (126) includes a longitudinal axis, a base defining an aperture therein for receiving the elongate shaft portion (122) of the bolt (114) therein and defining a plane that is angled with respect to the longitudinal axis.

6. The medical fastening system of any one of the preceding claims, wherein the superior surface (134) and the inferior surface (132) of the washer (116) are curved.

7. The medical fastening system of any one of the preceding claims, wherein the washer (116) includes a peripheral region (136) that defines a pair of opposed arcuate portions (140) and a pair of opposed flattened portions (142), the femoral component (128) includes a boss cavity (46) that defines the aperture (48) of the femoral component (128), the boss cavity (46) including a pair of opposed arcuate portions (52) and a pair of opposed flattened portions (54), the washer (116) being insertable into the boss cavity (46) in one of a first orientation and a second orientation, and once inserted being non-rotatable with respect to the femoral component (128).

8. The medical fastening system of any one of the preceding claims, wherein the aperture (144) of the washer (116) includes first and second lobes (146, 148) dimensioned to receive the elongated shaft portion (122) of the bolt (114) therethrough, the first and second lobes (146, 148) being defined by a neck portion (150) of the washer (116) having a diameter less than the diameter of the elongated shaft portion (122), wherein the aperture (48) in the femoral component (128) has an anterior portion, a central portion, and a posterior portion, and wherein the washer (116) is matable with the femoral component (128) to align the first lobe (146) of the washer (116) with one of the anterior portion and the posterior portion of the aperture (48) of the femoral component (128), and the second lobe portion (148) with the central portion of the aperture (48) of the femoral component (128).

## Patentansprüche

1. Medizinisches Befestigungssystem für eine modulare Knieprothese (102) mit:
einer femoralen Komponente (128) mit einer oberen Oberfläche (42), einer unteren Oberfläche (44) und einer sich dazwischen erstreckenden Öffnung (48), wobei die obere Oberfläche (42) im Gebrauch der Tibia zugewandt ist,
einer Scheibe (116) mit einer oberen Oberfläche (134), einer unteren Oberfläche (132) und einer sich dazwischen erstreckenden Öffnung (144), wobei die untere Oberfläche (132) der Scheibe (116) mit der oberen Oberfläche (42) der femoralen Komponente (128) in Eingriff bringbar ist, um eine Bewegung der Scheibe (116) durch die Öffnung (48) in der femoralen Komponente (128) hindurch zu verhindern, und
einem Bolzen (114), der einen Kopfabschnitt (120) aufweist, der mit der oberen Oberfläche (134) der Scheibe (116) in Eingriff bringbar ist, um eine Bewegung des Bolzens (114) durch die Öffnung (144) in der Scheibe (116) hindurch zu verhindern, und einem länglichen Schaftabschnitt (122), der sich von dem Kopfabschnitt (120) des Bolzens (114) erstreckt und der eine Länge aufweist, die ausreichend ist, um durch die Öffnung (144) in der Scheibe (116) und durch die Öffnung (48) in der femoralen Komponente (128) hindurch bis jenseits der unteren Oberfläche (44) der femoralen Komponente (128) zu ragen, wobei:
die Öffnung (144) der Scheibe (116) kleiner ist als die Öffnung (48) in der femoralen Komponente (128),
die Öffnung (48) in der femoralen Komponente (128) länglich ist und einen anterioren Abschnitt und einen posterioren Abschnitt aufweist,
die Scheibe (116) mit der femoralen Komponente (128) zusammenfügbar ist, um die Öffnung (144) der Scheibe (116) in Richtung des anterioren Abschnitts der Öffnung (48) der femoralen Komponente (128) zu positionieren, und
die Scheibe (116) mit der femoralen Komponente (128) zusammenfügbar ist, um die Öffnung (144) der Scheibe (116) in Richtung des posterioren Abschnitts der Öffnung der femoralen Komponente zu positionieren,
**dadurch gekennzeichnet, dass**
die Öffnung (144) in der Scheibe (116) nicht in der Scheibenmitte (116) angeordnet ist, wobei die zwei Öffnungspositionen erreicht werden, indem die Orientierung der Scheibe (116) geändert wird.

2. Medizinisches Befestigungssystem nach Anspruch 1, **dadurch gekennzeichnet, dass** das Befestigungssystem des Weiteren eine Zusatzkomponente (126) aufweist, die mit dem länglichen Schaftabschnitt (122) des Bolzens (114) in Eingriff bringbar ist.

3. Medizinisches Befestigungssystem nach Anspruch 2, **dadurch gekennzeichnet, dass** die Zusatzkomponente (126) ein Morsekegelschaft ist.

4. Medizinisches Befestigungssystem nach Anspruch 3, **dadurch gekennzeichnet, dass** der Morsekegelschaft (126) eine longitudinale Achse aufweist, eine Basis, in der eine Öffnung ausgebildet ist, um in dieser den länglichen Schaftabschnitt (122) des Bolzens (114) aufzunehmen, wobei die Basis eine Ebene bildet, die senkrecht zu der longitudinalen Achse ausgerichtet ist, und ein kegelstumpfartiges distales Ende.

5. Medizinisches Befestigungssystem nach Anspruch 3, **dadurch gekennzeichnet, dass** der Morsekegelschaft (126) eine longitudinale Achse aufweist, eine Basis, in der eine Öffnung ausgebildet ist, um in dieser den länglichen Schaftabschnitt (122) des Bolzens (114) aufzunehmen, wobei die Basis eine Ebene bildet, die zu der longitudinalen Achse abgewinkelt ist.

6. Medizinisches Befestigungssystem nach einem der vorhergehenden Ansprüche, **dadurch gekennzeichnet, dass** die obere Oberfläche (134) und die untere Oberfläche (132) der Scheibe (116) gekrümmt sind.

7. Medizinisches Befestigungssystem nach einem der vorhergehenden Ansprüche, **dadurch gekennzeichnet, dass** die Scheibe (116) eine Umfangsbereich (136) umfasst, der ein Paar von einander gegenüberliegenden gebogenen Abschnitten (140) und ein Paar von einander gegenüberliegenden abgeflachten Abschnitten (142) aufweist, wobei die femorale Komponente (128) einen Anschlusshohlraum (46) umfasst, der die Öffnung (48) der femoralen Komponente (128) bildet, wobei der Anschlusshohlraum (46) ein Paar von einander gegenüberliegenden gebogenen Abschnitten (52) und ein Paar von einander gegenüberliegenden abgeflachten Abschnitten (54) aufweist, wobei die Scheibe (116) in den Anschlusshohlraum (46) in entweder einer ersten Orientierung oder einer zweiten Orientierung einsetzbar ist und wobei die Scheibe, wenn sie eingesetzt ist, bezügl. der femoralen Komponente (128) nicht drehbar ist.

8. Medizinisches Befestigungssystem nach einem der vorhergehenden Ansprüche, **dadurch gekennzeichnet, dass** die Öffnung (144) der Scheibe (116) einen ersten und einen zweiten Öffnungsbereich (146, 148) aufweist, die so dimensioniert sind, dass sie den länglichen Schaftabschnitt (122) des Bolzens (114) durch diese hindurch aufnehmen, wobei die ersten und zweiten Öffnungsbereiche (146, 148) durch einen Kragenabschnitt (150) der Scheibe (116) ausgebildet sind, der einen Durchmesser aufweist, der kleiner ist als der Durchmesser des länglichen Schaftabschnittes (122), wobei die Öffnung (48) in der femoralen Komponente (128) einen anterioren Abschnitt, einen mittigen Abschnitt und einen posterioren Abschnitt aufweist und wobei die Scheibe (116) mit der femoralen Komponente (128) zusammenfügbar ist, um den ersten Öffnungsbereich (146) der Scheibe (116) mit entweder dem anterioren Abschnitt oder dem posterioren Abschnitt der Öffnung (48) der femoralen Komponente (128), und den zweiten Öffnungsbereichsabschnitt (148) mit dem mittigen Abschnitt der Öffnung (48) der femoralen Komponente (128) auszurichten.

## Revendications

1. Système de fixation médical pour une prothèse modulaire du genou (102), comprenant:
- un composant fémoral (128) ayant une surface supérieure (42), une surface inférieure (44) et une ouverture (48) s'étendant entre elles, la surface supérieure (42) faisant face au tibia lors de l'utilisation ;
- une rondelle (116) possédant une surface supérieure (134), une surface inférieure (132) et une ouverture (144) s'étendant entre elles, la surface inférieure (132) de la rondelle (116) entrant en contact avec la surface supérieure (42) du composant fémoral (128) afin d'empêcher tout mouvement de la rondelle (116) dans l'ouverture (48) du composant fémoral (128) ; et
- un boulon (114) comportant une partie tête (120) entrant en contact avec la surface supérieure (134) de la rondelle (116) afin d'empêcher tout mouvement du boulon (114) à travers l'ouverture (144) de la rondelle (116), et une partie arbre allongée (122) s'étendant de la partie tête (120) du boulon (114), laquelle partie arbre allongée (122) possède une longueur suffisante pour dépasser par l'ouverture (144) de la rondelle (116) et l'ouverture (48) du composant fémoral (128) au-delà de la surface inférieure (44) du composant fémoral (128) ;
dans lequel:
- l'ouverture (144) de la rondelle (116) est plus petite que l'ouverture (48) du composant fémoral (128) ;
- l'ouverture (48) du composant fémoral (128) est allongée et possède une partie antérieure et une partie postérieure ;
- la rondelle (116) vient s'adapter avec le composant fémoral (128) de manière à positionner l'ouverture (144) de la rondelle (116) vers la partie antérieure de l'ouverture (48) du composant fémoral (128) ; et
- la rondelle (116) vient s'adapter avec le composant fémoral (128) de manière à positionner l'ouverture (144) de la rondelle (116) vers la partie postérieure de l'ouverture du composant fémoral ;
**caractérisé en ce que** l'ouverture (144) de la rondelle (116) ne représente pas le centre de la rondelle (116), les deux positionnements de l'ouverture se faisant en changeant l'orientation de la rondelle (116).

2. Système de fixation médical, selon la revendication 1, comprenant en outre un composant supplémentaire (126) entrant en contact avec la partie arbre allongée (122) du boulon (114).

3. Système de fixation médical, selon la revendication 2, dans lequel le composant supplémentaire (126) consiste en une queue à cône Morse.

4. Système de fixation médical, selon la revendication 3, dans lequel la queue à cône Morse (126) possède un axe longitudinal, une base y définissant une ouverture afin d'y recevoir la partie arbre allongée (122) du boulon (114) et définissant un plan qui est perpendiculaire à l'axe longitudinal, ainsi qu'une extrémité distale se rétrécissant.

5. Système de fixation médical, selon la revendication 3, dans lequel la queue à cône Morse (126) possède un axe longitudinal, une base y définissant une ouverture afin d'y recevoir la partie arbre allongée (122) du boulon (114) et définissant un plan qui se trouve à un certain angle par rapport à l'axe longitudinal.

6. Système de fixation médical, selon l'une quelconque des revendications précédentes, dans lequel la surface supérieure (134) et la surface inférieure (132) de la rondelle (116) sont courbes.

7. Système de fixation médical, selon l'une quelconque des revendications précédentes, dans lequel : la rondelle (116) comprend une région périphérique (136) qui définit deux parties arquées opposées (140) et deux parties aplaties opposées (142) ; le composant fémoral (128) comprend une cavité de bossage (46) qui définit l'ouverture (48) du composant fémoral (128), la cavité de bossage (46) comprenant deux parties arquées opposées (52) et deux parties aplaties opposées (54), la rondelle (116) venant s'insérer dans la cavité de bossage (46) selon une première orientation ou selon une seconde orientation et, une fois insérée, ne peut tourner par rapport au composant fémoral (128).

8. Système de fixation médical, selon l'une quelconque des revendications précédentes, dans lequel l'ouverture (144) de la rondelle (116) comprend un premier et un second lobes (146, 148) dont les dimensions permettent de recevoir la partie arbre allongée (122) du boulon (114) au travers, les premier et second lobes (146, 148) étant définis par une partie col (150) de la rondelle (116) ayant un diamètre inférieur au diamètre de la partie arbre allongée (122), dans lequel l'ouverture (48) du composant fémoral (128) possède une partie antérieure, une partie centrale et une partie postérieure, et dans lequel la rondelle (116) vient s'adapter avec le composant fémoral (128) afin d'aligner le premier lobe (146) de la rondelle (116) avec la partie antérieure ou la partie postérieure de l'ouverture (48) du composant fémoral (128), et d'aligner la seconde partie lobe (148) avec la partie centrale de l'ouverture (48) du composant fémoral (128).
